# EUROPÄISCHE PATENTSCHRIFT

(11) **EP 0 696 607 B1**
(45) Veröffentlichungstag und Bekanntmachung des Hinweises auf die Patenterteilung: **01.04.1998**
(21) Anmeldenummer: 95112007.0
(22) Anmeldetag: 31.07.1995
(51) Int. Cl.: C08G 69/36, A61K 7/06

(54) **Sulfonatgruppentragende Polyamide und deren Verwendung in Haarfestigungsmitteln**
Sulphonate group containing polyamides and their use in hair-fixing agents
Polyamides contenant des groupes sulfonates et leur application dans des fixateurs pour cheveux

(30) Priorität: 08.08.1994 DE 4428003
(43) Veröffentlichungstag der Anmeldung: 14.02.1996
(73) Patentinhaber: BASF AKTIENGESELLSCHAFT, 67056 Ludwigshafen (DE)
(72) Erfinder: Fisch, Herbert, Dr., D-67157 Wachenheim (DE); Stein, Stefan, Dr., D-55291 Saulheim (DE); Sanner, Axel, Dr., D-67227 Frankenthal (DE); Sperling-Vietmeier, Karin, Dr., D-67433 Neustadt (DE); Breitenbach, Jörg, Dr., D-68199 Mannheim (DE)

(56) Entgegenhaltungen:
- DE-A- 1 570 549
- FR-A- 2 256 195
- LU-A- 68 507
- US-A- 3 296 204
- US-A- 5 186 762

## Beschreibung

Die vorliegende Erfindung betrifft die Verwendung von sulfonatgruppentragenden Polyamiden, welche erhältlich sind aus
A₁) 20 bis 99 mol-% einer Monoaminocarbonsäure mit 2 bis 12 C-Atomen oder deren Lactam,
A₂) 0,5 bis 40 mol-% eines Diamins mit 2 bis 18 C-Atomen,
A₃) 0,5 bis 25 mol-% einer sulfonatgruppentragenden Dicarbonsäure mit 4 bis 12 C-Atomen, und
A₄) 0 bis 35 mol-% einer weiteren Dicarbonsäure mit 2 bis 16 C-Atomen,
als Filmbildner in Haarfestigungsmitteln.

Weiterhin betrifft die Erfindung neue sulfonatgruppenhaltige Polyamide, deren Dispersionen sowie haarkosmetische Zubereitungen.

Zum Festigen, Strukturverbessern und Formgeben von Haaren werden im allgemeinen synthetische Polymere verwendet. Bewährte Polymere sind beispielsweise solche auf Basis von Vinylpyrrolidon und Vinylacetat, die üblicherweise in Form alkoholischer oder wäßrigalkoholischer Lösungen appliziert werden.

Die Lösungen dieser Materialien umhüllen die behandelten Haare mit einem Film, der je nach eingesetztem Polymertyp festigend, strukturverbessernd, formgebend, glanzverbessernd, glättend und antistatisch wirken kann. Die auf den Haaren gebildeten Filme sollen einerseits feuchtigkeitsbeständig sein, d.h. das Haar soll auch bei hoher Luftfeuchtigkeit nicht verkleben und die Form verlieren, andererseits sollen sich diese Filme bei der Reinigung der Haare leicht mit einer wäßrigen Tensidlösung auswaschen lassen.

Aufgrund der ständig wachsenden Forderung nach umweltverträglicheren Einsatzstoffen soll der Gehalt an flüchtigen organischen Bestandteilen (volatile organic compounds - VOC) auch in kosmetischen Zubereitungen wie beispielsweise Haarspray möglichst niedrig gehalten werden bzw. gesenkt werden. Das bedeutet, daß der Alkohol als Lösungsmittel zunehmend durch Wasser ersetzt werden soll.

Bei Verwendung herkömmlicher Haarfestigungspolymere kann dies jedoch Schwierigkeiten bereiten, da bei höheren Wassergehalten Viskosität und Sprühverhalten der Haarfestigungsmittel und die Trocknung der Polymerfilme nicht befriedigend sind.

Aus der US-A 3 296 204 ist die Herstellung von sulfonathaltigen Polyamiden durch Kondensation sulfonierter aromatischer Dicarbonsäuren, deren Sulfonsäuregruppe als Alkalisalz vorliegt, mit Diaminen bekannt.

In der DE-C 23 08 266 wird die Verwendung solcher sulfonierter Polyamide zur Herstellung von Polyamidgarnen beschrieben.

Die US-A 5 158 762 betrifft wäßrige Haarsprayzusammensetzungen, in denen als Filmbildner Polymerblends aus einem wasserlöslichen Polymer und einem sulfonatgruppenhaltigen Polyester oder Polyesteramid eingesetzt werden.

Aufgabe der vorliegenden Erfindung war es, Polymere für den Einsatz als Filmbildner in Haarfestigungsmitteln zu finden, die erhöhte Wasseranteile in den entsprechenden kosmetischen Zubereitungen ermöglichen, ohne daß eine Viskositätserhöhung bewirkt oder andere anwendungstechnische Eigenschaften wie das Sprühverhalten negativ beeinflußt werden. Nach der Applikation auf das Haar sollen außerdem klebfreie, klare Filme mit guter Festigungswirkung und guter Auswaschbarkeit erhalten werden.

Demgemäß wurden die Verwendung der eingangs definierten sulfonattragenden Polyamide, neue Polyamide, deren Dispersionen und haarkosmetische Zubereitungen, enthaltend sulfonattragende Polyamide, gefunden.

Erfindungsgemäß eignen sich als Filmbildner sulfonatgruppentragende Polyamide, die erhältlich sind aus
A₁) 20 bis 99 mol-% vorzugsweise 30 bis 94 mol-%, einer Monoaminocarbonsäure mit 2 bis 12 C-Atomen oder deren Lactame oder Mischungen davon,
A₂) 0,5 bis 40 mol-%, vorzugsweise 7 bis 35 mol-%, eines Diamins mit 2 bis 18 C-Atomen,
A₃) 0,5 bis 25 mol-%, vorzugsweise 2,6 bis 20 mol-%, besonders bevorzugt 10 bis 19 mol-%, einer sulfonatgruppentragenden Dicarbonsäure mit 4 bis 12 C-Atomen, und
A₄) 0 bis 35 mol-%, vorzugsweise 5 bis 30 mol-%, einer weiteren Dicarbonsäure mit 2 bis 16 C-Atomen,
wobei die Summe der molaren Anteile der Monomeren A₃) und A₄) dem molaren Anteil des Monomeren A₂) entspricht.

Als Monomere A₁) eignen sich die für die Herstellung von Polyamiden bekannten Monoaminocarbonsäuren oder deren Lactame wie beispielsweise ω-Aminoundecansäure, ε-Caprolactam, Laurinlactam, Capryllactam oder Önantholactam.

Als Monomere A₂) kommen aliphatische, cycloaliphatische oder aromatische Diamine in Betracht. Geeignete Diamine sind beispielsweise Alkylendiamine oder Cycloalkyldiamine wie 1,5-Pentandiamin, 4,4'-Diaminodicyclohexylmethan, 2,2'-(4,4'-Diaminodicyclohexyl)propan, 3,3'-Dimethyl-4,4'-diaminodicyclohexylmethan oder, vorzugsweise, Hexamethylendiamin. Weiterhin eignen sich auch Piperazin, 2,2,4-Trimethylhexamethylendiamin, 2-Butyl-2-ethyl-1,5-pentandiamin oder 4,7-Dioxadecan-1,10-diamin.

Als sulfonatgruppentragende Monomere A3) kommen solche Verbindungen in Betracht, in denen die Sulfonatgruppe als Lithium-, Natrium-, Kalium- oder Ammoniumsalz vorliegt. Geeignete sulfonatgruppentragende Monomere sind Salze von aliphatischen oder aromatischen Dicarbonsäuren wie beispielsweise Sulfobernsteinsäure oder 5-Sulfopropoxyisophthalsäure. Bevorzugt wird das Natriumsalz der 5-Sulfo-isophthalsäure eingesetzt.

Geeignete Monomere A₄) sind beispielsweise aliphatische Dicarbonsäuren wie Sebacinsäure, Azelainsäure, Dodecandicarbonsäure oder bevorzugt Adipinsäure oder Sebacinsäure. Geeignete aromatische Dicarbonsäuren sind beispielsweise Isophthalsäure oder Terephthalsäure, die auch substituiert sein können wie beispielsweise die 3-tert.-Butyl-isophthalsäure, weiterhin 3,3'- oder 4,4'-Diphenyl-dicarbonsäure, 3,3'- oder 4,4'-Diphenylmethan-di-carbonsäure, 3,3'- oder 4,4'-Diphenylsulfon-dicarbonsäure, 1,4- oder 2,6-Naphthalindicarbonsäure oder 2-Phenoxyterephthalsäure.

Selbstverständlich gilt für alle Monomerengruppen, daß auch Gemische der jeweiligen Monomeren eingesetzt werden können.

Neue Polyamide sind erfindungsgemäß solche, die erhältlich sind aus
a₁) 30 bis 94,8, vorzugsweise 52 bis 85 mol-%, insbesondere 60 bis 80 mol-% der Monomeren A₁),
a₂) 2,6 bis 37, vorzugsweise 5,9 bis 35,5 mol-% der Monomeren A₂),
a₃) 2,6 bis 7, vorzugsweise 2,9 bis 6,5 mol-% der Monomeren A₃) und
a₄) 0 bis 30, vorzugsweise 3 bis 29 mol-%, insbesondere 3 bis 25 mol-% der Monomeren A₄).

Die Herstellung der sulfonatgruppentragenden Polyamide kann auf an sich bekannte Weise erfolgen.

Als bevorzugte Herstellweise sei der Batch-Prozeß (diskontinuierliche Herstellweise) genannt. Dabei wird die wäßrige Monomerlösung innerhalb von 0,5 bis 3 h in einem Autoklaven auf Temperaturen von 240 bis 300°C erhitzt, wobei ein Druck von 10 bis 50 bar, insbesondere 15 bis 30 bar, erreicht wird, der durch Entspannen von überschüssigem Wasserdampf bis zu 4 h konstant gehalten wird. Danach entspannt man den Autoklgyen bei konstanter Temperatur innerhalb eines Zeitraumes von 0,5 bis 3 h auf Normaldruck. Anschließend wird die Polymerschmelze aus dem Autoklaven ausgefahren, über Luft- oder Stickstoffkühlung abgekühlt und anschließend granuliert.

Das so erhaltene Copolyamid hat in der Regel eine Viskositätszahl zwischen 25 und 110 ml/g, bevorzugt von 30 bis 80 ml/g, gemessen an einer 0,5 gew.-%igen Lösung in 96 %iger Schwefelsäure.

Zur Herstellung der erfindungsgemäßen Polyamiddispersionen können die Polyamidgranulate in 30 bis 99, vorzugsweise 60 bis 90 Gew.-%, bezogen auf die Polymermenge, an Wasser durch intensives Rühren dispergiert werden. Die Herstellung der Dispersionen erfolgt üblicherweise bei 25°C, kann aber auch bei Temperaturen bis zu 80°C durchgeführt werden. Nach Zugabe des Wassers werden die Mischungen vorzugsweise noch 0,5 bis 3 h gerührt, wobei eine vollständige Dispersion der Polyamide erfolgt. Die so hergestellten Dispersionen weisen Feststoffgehalte von 1 bis 70, vorzugsweise 10 bis 40 Gew.-% auf. Die Lichtdurchlässigkeit (bestimmt mittels eines Vis-Spektrometers der Fa. Beckmann) liegt bei 50 bis 99 %, vorzugsweise > 60 %. Die Partikelgrößen können im Bereich von 40 bis 120 nm liegen.

Vorteilhaft an den neuen sulfonatgruppentragenden Polyamiden ist, daß sie sich problemlos in Wasser zu Dispersionen mit höheren Feststoffgehalten verarbeiten lassen.

Sulfonatgruppentragende Polyamide, welche weniger als 2,6 mol-% eines Salzes einer sulfonatgruppentragenden Dicarbonsäure enthalten, lassen sich nicht mehr direkt zu wäßrigen Dispersionen verarbeiten. Beträgt der Anteil des sulfonatgruppentragenden Monomeren mehr als 7 mol-%, lassen sich Dispersionen zwar prinzipiell herstellen, jedoch bedingt durch die hohe Viskosität nur noch bis zu Feststoffgehalten unter 10 Gew.-%.

Damit eignen sich die erfindungsgemäßen Polyamide vor allem für solche Haarfestigungsmittel, die einen besonders niedrigen VOC-Gehalt aufweisen sollen, also überwiegend wasserbasierend sind. Bevorzugt kann man sie in Haarfestigungsmitteln mit VOC-Werten < 55 Gew.-% einsetzen. Wegen der guten Selbstdispergierbarkeit der erfindungsgemäßen Polyamide kann auf größere Anteile organischer Lösungsmittel verzichtet werden.

Jedoch eignen sich erfindungsgemäß als Filmbildner in Haarfestigungsmitteln auch solche sulfonatgruppenhaltigen Polyamide, die durch Einsatz von geringeren oder höheren Anteilen der sulfonatgruppentragenden Monomere erhältlich sind, und zwar vor allem dann, wenn die Haarfestigungsmittel VOC-Werte von > 55 % aufweisen können. Die Haarfestigungsmittel stellen in einem solchen Fall alkoholische oder wäßrig-alkoholische Lösungen der sulfonatgruppenhaltigen Polyamide oder auch Lösungen der Polyamide in anderen hierfür geeigneten organischen Lösungsmitteln dar. Aufgrund der besseren Verdampfbarkeit des organischen Lösungsmittels im Vergleich zu Wasser können diese Lösungen auch mit einem geringeren Feststoffgehalt, beispielsweise im Bereich von 1 bis 10 Gew.-%, als Haarfestigungsmittel eingesetzt werden.

Erfindungsgemäß können die sulfonattragenden Polyamide sowohl als alleinige Filmbildner in Haarfestigungsmitteln als auch als Mischungen mit herkömmlichen Haarfestigungspolymeren eingesetzt werden. Als herkömmliche Haarfestigungspolymere eignen sich beispielsweise anionische Polymere wie Acrylsäure- oder Methacrylsäure-Homopolymere oder -Copolymere, Copolymerisate aus Acrylsäure und Acrylamiden und Copolymere auf der Basis von Alkylvinylethern und Maleinsäuremonoalkylestern, amphotere Polymere wie Copolymerisate aus Octylacrylamid, Acrylat und Butylaminoethylmethacrylat sowie nichtionische Polymere wie Vinylpyrrolidon-Homopolymere, Vinylpyrrolidon-Vinylacetat-Copolymere und Copolymerisate aus Vinylpyrrolidon, Vinylacetat und Vinylpropionat. Das Verhältnis von sulfonatgruppenhaltigen Polyamiden zu den herkömmlichen Haarfestigungspolymeren kann entsprechend den gewünschten anwendungstechnischen Eigenschaften gewählt werden.

Weiterhin können die Haarfestigungsmittel übliche Hilfsstoffe wie beispielsweise Tenside, Emulgatoren oder Duftstoffe enthalten.

Als Haarfestigungsmittel geeignete Formulierungen können beispielsweise wie folgt zusammengesetzt sein (% = Gew.-%):
1)

| | |
|---|---|
| 5 % | eines Polyamids |
| 0,2 % | einer Mischung aus Parfümöl und Emulgator im Mengenverhältnis 1:3 |
| 10 % | Ethanol |
| 84,8 % | Wasser |

2)

| | |
|---|---|
| 3,5 % | eines Polyamids |
| 1,5 % | eines Copolymers aus 60 Gew.-% N-Vinylpyrrolidon und 40 Gew.-% Vinylacetat |
| 0,2 % | Parfümöl/Emulgator |
| 94,8 % | Wasser |

Anstelle eines NVP/VA-Copolymers können beispielsweise auch 1,5 % eines Terpolymers auf Acrylatbasis verwendet werden.

Bei Anwendung der erfindungsgemäßen Haarfestigungsmittel werden klare, gut trocknende Filme erhalten, die ein angenehmes Hautgefühl vermitteln, gute Festigungswirkung und gute Auswaschbarkeit zeigen.

### Beispiele 1 bis 6 und Vergleichsbeispiele 1 und 2 (Tabelle 1)

In einem 5-1-Laborautoklaven wurden 2 kg einer Monomerenmischung der in Tabelle 1 angegebenen Zusammensetzung in 1500 ml Wasser vorgelegt. Der Autoklav wurde innerhalb 1 h auf 280°C aufgeheizt, wobei der resultierende Druck von ca. 20 bar durch Entspannen überschüssigen Wasserdampfes konstant gehalten wurde. Druck und Temperatur wurden eine weitere Stunde unverändert. Anschließend wurde der Autoklav unter Beibehaltung der Temperatur von 280°C innerhalb von 1 h auf Normaldruck entspannt. Anschließend wurde 2 h lang im Stickstoffstrom nachkondensiert. Die Schmelze wurde danach über eine Düse ausgefahren, in einem Luftbett gekühlt und granuliert.

Jeweils 400 g des Granulats wurden in 600 g Wasser bei Raumtemperatur unter Rühren dispergiert.

An den Granulaten wurden folgende Prüfungen durchgeführt:
a) Granulat vor der Dispergierung: Bestimmung der Viskositätszahl (VZ) in Anlehnung an DIN 53 246 (0,5 %ige Lösung des Copolyamids in 96 %iger H₂SO₄).
b) Bestimmung der Glasübergangstemperatur (Tg) und des Schmelzbereiches mittels **D**ifferential **S**canning **C**alorimetrie (DSC 5000 der Fa. Mettler) bei 20°C/min Aufheizrate.

Die Charakterisierung der Dispersionen erfolgte nach folgenden Methoden:
a) Bestimmung der Lichtdurchlässigkeit (LD-Messung, 0,01 %ige Lösungen in Wasser, Vergleich: reines Wasser: LD=100)
b) Bestimmung der Viskosität im Rotationsviskosimeter (Modell: RV 20; Fa. Haake) bei einer Scherung von 500 s⁻¹; T=23°C
c) Bestimmung der Partikelgröße im Nanosizer (Modell: Autosizer 2C; Fa. Malvern)

**Tabelle 1:**

| Zusammensetzung und Eigenschaften gemäß den Beispielen 1 bis 6 (Angaben in mol-%) | | | | | | |
|---|---|---|---|---|---|---|
| Komponente | Beisp. 1 | Beisp. 2 | Beisp. 3 | Beisp. 4 | Beisp. 5 | Beisp. 6 |
| A1 ε-Caprolactam | 50 | 60 | 70 | 75 | 60 | 60 |
| A2 1,6-Hexamethylendiamin | 25 | 20 | 15 | 12,5 | 20 | 20 |
| A3 5-Sulfoisophthalsäure-Natrium-Salz | 3 | 3 | 3 | 3 | 5 | 3 |
| A4 Isophthalsäure | 22 | 17 | 12 | 9,5 | 15 | 17 |
| B [%] Wasser | 60 | 60 | 60 | 60 | 70 | 60 |
| VZ [ml/g] | 71,5 | 79 | 87 | 112 | 63 | 85 |
| η [mPas] | 98 | 122 | 140 | 110 | 200 | 110 |
| LD [%] | 96 | 99 | 86 | 66 | 100 | 87 |
| Tg [°C] | 92 | 98 | 69 | 74 | 98 | 59 |
| Teilchengröße [nm] | 77 | 48 | 107 | 140 | 41 | 60 |

### Beispiele 7 bis 12

2 kg einer Monomerenmischung der in Tabelle 2 angebenen Zusammensetzung wurden in 1500 ml Wasser gelöst und unter den in Tabelle 2 aufgeführten Bedingungen in der Schmelze kondensiert.

**Tabelle 2:**

| Zusammensetzungen der Polymeren gemäß den Beispielen 7 bis 12 (Angaben in mol-%) | | | | | | | | |
|---|---|---|---|---|---|---|---|---|
| Beispiel Nr. | 7 | 8 | 9 | 10 | 11 | 12 | 13 | 14 |
| ε-Caprolactam | 20 | 20 | 20 | 20 | 20 | 20 | 32 | 50 |
| Adipinsäure | - | - | - | - | 34 | 20 | - | - |
| 4,4'-Bis(diamino-3,3'-dimethylcyclohexyl)-methan | 24 | 24 | 24 | 40 | - | - | - | - |
| Hexamethylendiamin | 16 | 16 | 16 | - | 40 | 40 | 34 | 25 |
| 5-Sulfoisophthalsäure-Natrium-Salz | 24 | 24 | 12 | 12 | 6 | 20 | 17 | 8 |
| Isophthalsäure | 16 | 16 | 28 | 28 | - | - | 17 | 17 |
| Temperatur [°C] | 270 | 250 | 270 | 270 | 270 | 270 | 270 | 270 |
| Druck [bar] | 20 | 8 | 20 | 20 | 20 | 20 | 20 | 20 |

### Formulierungsbeispiele

I)

| | |
|---|---|
| 10 Gew.-% | Polyamid gemäß Beispiel 13 |
| 0,4 Gew.-% | einer Mischung aus Parfümöl und Emulgator ¹) im Gewichtsverhältnis 1:3 |
| 89,6 Gew.-% | Wasser |

| | |
|---|---|
| ¹) ethoxyliertes hydriertes Rizinusöl, hergestellt mit 40 mol Ethylenoxid | |

II)

| | |
|---|---|
| 10 Gew.-% | Polyamid gemäß Beispiel 14 ansonsten wie in Formulierung I) |

### Anwendungstechnische Prüfung

### Initial Curl Droop Methode:

Bei der Initial Curl Droop Methode werden 2 g einer 15,5 cm langen Haarsträhne zweimal mit Texapon NSO-Lösung (Natriumlaurylethersulfat) mit 10 % Feststoffanteil gewaschen, anschließend wird mit Wasser gespült und für 15 min bei 40°C in 50 %iges Ethanol gegeben. Die überschüssige Flüssigkeit wird herausgepreßt und das Haar wird um ein Plexiglasrohr (12 mm Durchmesser) gewickelt. Anschließend wird 1 h bei 65 bis 70°C getrocknet. Nach 15 min Abkühlen bei Raumtemperatur wird das Haar abgewickelt. Die Locke wird an einem Ende aufgehängt und die Lockenlänge (Lₒ) gemessen. Anschließend wird die Haarsträhne 4 s lang aus ca. 10 cm Entfernung mit dem Haarspray besprüht, wobei die Locke gedreht wird. Danach wird die Locke erneut aufgehängt und in die Klimakammer gegeben. Im zeitlichen Abstand wird die Lockenlänge (Lₜ) gemessen. Der Initial Curl Droop ergibt sich zu: L - Lₜ /(L - Lₒ) * 100

| | Formulierung I | Formulierung II |
|---|---|---|
| Initial Curl Droop [%] | 79 | 72 |
| Festigung | gut | gut |
| Auswaschbarkeit | gut | gut |

## Patentansprüche

1. Verwendung von sulfonatgruppentragenden Polyamiden, erhältlich aus
A₁) 20 bis 99 mol-% einer Monoaminocarbonsäure mit 2 bis 12 C-Atomen oder deren Lactam,
A₂) 0,5 bis 40 mol-% eines Diamins mit 2 bis 18 C-Atomen,
A₃) 0,5 bis 25 mol-% einer sulfonatgruppentragenden Dicarbonsäure mit 4 bis 12 C-Atomen, und
A₄) 0 bis 35 mol-% einer weiteren Dicarbonsäure mit 2 bis 16 C-Atomen,
als Filmbildner in Haarfestigungsmitteln.

2. Wäßrige Haarfestigungsmittel, enthaltend als Filmbildner sulfonatgruppentragende Polymere, erhältlich aus
A₁) 20 bis 99 mol-% einer Monoaminocarbonsäure mit 2 bis 12 C-Atomen oder deren Lactam,
A₂) 0,5 bis 40 mol-% eines Diamins mit 2 bis 18 C-Atomen,
A₃) 0,5 bis 25 mol-% einer sulfonatgruppentragenden Dicarbonsäure mit 4 bis 12 C-Atomen, und
A₄) 0 bis 35 mol-% einer weiteren Dicarbonsäure mit 2 bis 16 C-Atomen.

3. Haarfestigungsmittel nach Anspruch 2, enthaltend zusätzlich weitere Haarfestigungspolymere.

4. Wasserdispergierbare, sulfonatgruppentragende Polyamide, erhältlich aus
a₁) 30 bis 94,8 mol-% einer Monoaminocarbonsäure mit 2 bis 12 C-Atomen oder deren Lactam,
a₂) 2,6 bis 37 mol-% eines Diamins mit 2 bis 18 C-Atomen,
a₃) 2,6 bis 7 mol-% einer sulfonatgruppentragenden Dicarbonsäure mit 4 bis 12 C-Atomen und
a₄) 0 bis 30 mol-% einer weiteren Dicarbonsäure mit 2 bis 16 C-Atomen.

5. Wäßrige Dispersionen, enthaltend 1 bis 70 Gew.-% eines Polyamids gemäß Anspruch 4.

## Claims

1. The use of sulfonate-bearing polyamides, obtainable from
A₁) from 20 to 99 mol% of a monoaminocarboxylic acid having 2 to 12 C atoms or its lactam,
A₂) from 0.5 to 40 mol% of a diamine having 2 to 18 C atoms,
A₃) from 0.5 to 25 mol% of a sulfonate-bearing dicarboxylic acid having 4 to 12 C atoms, and
A₄) from 0 to 35 mol% of a further dicarboxylic acid having 2 to 16 C atoms,
as film-forming agents in hairsetting compositions.

2. An aqueous hairsetting composition, containing as film-forming agent sulfonate-bearing polymers obtainable from
A₁) from 20 to 99 mol% of a monoaminocarboxylic acid having 2 to 12 C atoms or its lactam,
A₂) from 0.5 to 40 mol% of a diamine having 2 to 18 C atoms,
A₃) from 0.5 to 25 mol% of a sulfonate-bearing dicarboxylic acid having 4 to 12 C atoms, and
A₄) from 0 to 35 mol% of a further dicarboxylic acid having 2 to 16 C atoms.

3. A hairsetting composition as claimed in claim 2, additionally containing further hairsetting polymers.

4. A water-dispersible, sulfonate-bearing polyamide, obtainable from
a₁) from 30 to 94.8 mol% of a monoaminocarboxylic acid having 2 to 12 C atoms or its lactam,
a₂) from 2.6 to 37 mol% of a diamine having 2 to 18 C atoms,
a₃) from 2.6 to 7 mol% of a sulfonate-bearing dicarboxylic acid having 4 to 12 C atoms and
a₄) from 0 to 30 mol% of a further dicarboxylic acid having 2 to 16 C atoms.

5. An aqueous dispersion, containing from 1 to 70% by weight of a polyamide as claimed in claim 4.

## Revendications

1. Utilisation de polyamides portant des groupes sulfonate, obtenus à partir de
A₁) 20 à 99 mol % d'un acide monoaminocarboxylique en C2-C12 ou de son lactame,
A₂) 0,5 à 40 mol % d'une diamine en C2-C18,
A₃) 0,5 à 25 mol % d'un acide dicarboxylique en C4-C12 portant des groupes sulfonate et
A₄) 0 à 35 mol % d'un autre acide dicarboxylique en C2-C16,
en tant qu'agents filmogènes dans des produits fixateurs de la chevelure.

2. Produit aqueux pour fixation de la chevelure, contenant, en tant qu'agents filmogènes, des polymères portant des groupes sulfonate obtenus à partir de
A₁) 20 à 99 mol % d'un acide monoaminocarboxylique en C2-C12 ou de son lactame,
A₂) 0,5 à 40 mol % d'une diamine en C2-C18,
A₃) 0,5 à 25 mol % d'un acide dicarboxylique en C4-C12 portant des groupes sulfonate et
A₄) 0 à 35 mol % d'un autre acide dicarboxylique en C2-C16.

3. Produit fixateur de la chevelure selon la revendication 2, contenant en outre d'autres polymères fixateurs de la chevelure.

4. Polyamides portant des groupes sulfonate, dispersables dans l'eau, obtenus à partir de
a₁) 30 à 94,8 mol % d'un acide monoaminocarboxylique en C2-C12 ou de son lactame,
a₂) 2,6 à 37 mol % d'une diamine en C2-C18,
a₃) 2,6 à 7 mol % d'un acide dicarboxylique en C4-C12 portant des groupes sulfonate et
a₄) 0 à 30 mol % d'un autre acide dicarboxylique en C2-C16.

5. Dispersions aqueuses contenant 1 à 70% en poids d'un polyamide selon la revendication 4.
